# EUROPÄISCHE PATENTSCHRIFT

(11) **EP 2 200 976 B1**
(45) Veröffentlichungstag und Bekanntmachung des Hinweises auf die Patenterteilung: **23.11.2011**
(21) Anmeldenummer: 08804005.0
(22) Anmeldetag: 11.09.2008
(51) Int. Cl.: C07C 263/10

(54) **VERFAHREN ZUR HERSTELLUNG VON ISOCYANATEN**
PROCESS FOR PREPARING ISOCYANATES
PROCÉDÉ DE FABRICATION D'ISOCYANATES

(30) Priorität: 19.09.2007 EP 07116729
(43) Veröffentlichungstag der Anmeldung: 30.06.2010
(73) Patentinhaber: BASF SE, 67056 Ludwigshafen (DE)
(72) Erfinder: KNÖSCHE, Carsten, 67150 Niederkirchen (DE); WÖLFERT, Andreas, 74906 Bad Rappenau (DE); MATTKE, Torsten, 67251 Freinsheim (DE); STROEFER, Eckhard, 68163 Mannheim (DE); BLANKERTZ, Heinrich-Josef, 67147 Forst (DE); GEISSLER, Bernhard, 67281 Kirchheim (DE)
(86) Internationale Anmeldenummer: PCT/EP2008/062038
(87) Internationale Veröffentlichungsnummer: WO 2009/037179

(56) Entgegenhaltungen:
- EP-A- 0 570 799
- EP-A- 1 078 918
- WO-A-2005/123665
- WO-A-2007/014936

## Beschreibung

Die vorliegende Erfindung betrifft ein Verfahren zur Herstellung von Diisocyanaten aus Diaminen und Phosgen in der Gasphase.

Die Phosgenierung von Aminen zu Isocyanaten in der Gasphase ist bekannt, beispielsweise - neben zahlreichen anderen Dokumenten - aus EP 570799, EP 749 958 B1 und EP 1078918 B1.

WO 2007/014936 A2 beschreibt ein Verfahren zur Herstellung von Diisocyanaten durch Umsetzung von Diaminen mit stöchiometrischem Überschuß an Phosgen mit einem phosgenhaltigen Gasstrom bei einer Temperatur von 200 °C bis zu 600 °C.

In diesem beschriebenen Verfahren wird das Phosgen einer flüssigen Vorlage entnommen.

In den bekannten Gasphasenphosgenierungsverfahren wird dabei Phosgen vor Einsatz in das Verfahren verdampft. Dies erfordert eine Lagerung von flüssigem Phosgen als Bevorratung für den Einsatz in das Verfahren und die Zufuhr von Energie zum Verdampfen.

Aufgrund der hohen Toxizität des Phosgen ist man aus Sicherheitsgründen bestrebt, die Bevorratung von Phosgen und die Menge an Phosgen, die sich jeweils in den Verfahrensstufen befindet (hold-up), so gering wie möglich zu halten. Diesem Bestreben läuft entgegen, daß in der Umsetzung von Amin das Phosgen überstöchiometrisch eingesetzt wird, so daß reaktionsbedingt beträchtliche Mengen Phosgen zurückgeführt werden müssen.

Aufgabe der vorliegenden Erfindung war es, ein Verfahren zur Herstellung von Diisocyanaten bereitzustellen, mit dem die Menge an Phosgen in den einzelnen Verfahrensstufen der Diisocyanatherstellung verringert werden kann.

Die Aufgabe wurde gelöst durch ein Verfahren zur Herstellung von Diisocyanaten durch Umsetzung der korrespondierenden Diamine mit Phosgen im stöchiometrischen Überschuß von Phosgen in mindestens einer Reaktionszone, umfassend zumindest die Verfahrensstufen
- Vermischen (V) mindestens eines gasförmiges Phosgen enthaltenden Stromes mit mindestens einem gasförmiges Diamin enthaltenden Strom und Umsetzung dieses Gemisches bei einer Temperatur von 200 bis 600 °C in der mindestens einer Reaktionszone (VI),
- Vermischung (VII) des entstehenden Reaktionsgemischs, das im wesentlichen aus Isocyanat, Phosgen und Chlorwasserstoff besteht, mit mindestens einer Flüssigkeit unter Absenkung der Temperatur auf 100 bis 200 °C, so daß das im Reaktionsgemisch enthaltene Isocyanat durch Kondensation zumindest teilweise in die Flüssigkeit übergeht, während Phosgen und Chlorwasserstoff im wesentlichen vollständig in der Gasphase verbleiben (Quench),
- (VIII) Aufarbeitung des kondensierten Isocyanats unter Verdampfung des in der kondensierten Phase verbliebenen Phosgens und Chlorwasserstoffs,
- (IX) Auftrennung der vereinigten gasförmigen im wesentlichen Chlorwasserstoff und Phosgen enthaltenden Ströme in einen überwiegend Phosgen und einen überwiegend Chlorwasserstoff enthaltenden Strom,
- Rückführung des so erhaltenen überwiegend Phosgen enthaltenden Stromes in die Vermischung (V),
in dem das in den Verfahrensstufen befindliche Phosgen zu mindestens 75 Gew% gasförmig vorliegt.

Bei der Gasphasenphosgenierung ist es erfindungsgemäß anzustreben, daß sämtliche im Reaktionsverlauf auftretenden Verbindungen, also Edukte (Diamin und Phosgen), Zwischenprodukte (insbesondere die intermediär entstehenden Mono- und Dicarbamoylchloride), Endprodukte (Diisocyanat, Chlorwasserstoff), sowie gegebenenfalls zudosiertes Inertmedium, unter den Reaktionsbedingungen in der Gasphase verbleiben.

Sollten diese oder andere Komponenten sich aus der Gasphase z. B. an der Reaktorwand oder anderen Apparatebauteilen abscheiden, so kann durch diese Abscheidungen der Wärmeübergang oder die Durchströmung der betroffenen Bauteile unerwünscht verändert werden.

Gemäß dem erfindungsgemäßen Verfahren erfolgt die Umsetzung von Phosgen mit Diamin in der Gasphase. Unter Umsetzung in der Gasphase ist zu verstehen, dass die Eduktströme im gasförmigen Zustand miteinander reagieren.

Diisocyanate, die mit dem erfindungsgemäßen Verfahren hergestellt werden können, können aromatische, cycloaliphatische oder aliphatische sein.

Cycloaliphatische Isocyanate sind solche, die mindestens ein cycloaliphatisches Ringsystem enthalten.

Aliphatische Isocyanate sind solche, die ausschließlich Isocyanatgruppen aufweisen, die an gerade oder verzweigte Ketten gebunden sind. Diese können auch aromatische Ringsysteme enthalten, solange an diese nicht Isocyanatgruppen gebunden sind.

Aromatische Isocyanate sind solche, die mindestens eine an mindestens ein aromatisches Ringsystem gebundene Isocyanatgruppe aufweisen.

(Cyclo)aliphatische Isocyanate steht im Rahmen dieser Anmeldung kurz für cycloaliphatische und/oder aliphatische Isocyanate.

Beispiele für aromatische Diisocyanate sind bevorzugt solche mit 6 - 20 C-Atomen, beispielsweise monomeres Methylen-di(phenylisocyanat (MDI), 2,4- und/oder 2,6-Toluylendiisocyanat (TDI) und Naphtyldiisocyanat (NDI).

Diisocyanate sind bevorzugt (cyclo)aliphatische Diisocyanate besonders bevorzugt (cyclo)aliphatische Diisocyanate mit 4 bis 20 C-Atomen.

Beispiele für übliche Diisocyanate sind aliphatische Diisocyanate wie Tetramethylendiisocyanat, Pentamethylendiisocyanat (1,5-Diisocyanatopentan), Hexamethylendiisocyanat (1,6-Diisocyanatohexan), 2-Methylpentan-1,5-diisocyanat, 1,8-Octamethylendiisocyanat, 1,10-Decamethylendiisocyanat, 1,12-Dodecamethylendiisocyanat, 1,14-Tetradecamethylendiisocyanat, Derivate des Lysindiisocyanates, Tetramethylxylylendiisocyanat (TMXDI), Trimethylhexandiisocyanat oder Tetramethylhexandiisocyanat, sowie 3 (bzw. 4), 8 (bzw. 9)-Bis(isocyanatomethyl)-tricyclo[5.2.1.0^{2.6}]decan-Isomerengemische, sowie cycloaliphatische Diisocyanate wie 1,4-, 1,3- oder 1,2-Diisocyanatocyclohexan, 4,4'- oder 2,4'-Di(isocyanatocyclohexyl)methan, 1-Isocyanato-3,3,5- trimethyl-5-(isocyanatomethyl)cyclohexan (Isophorondiisocyanat), 1,3- oder 1,4-Bis(isocyanatomethyl)cyclohexan, 2,4-, oder 2,6-Diisocyanato-1-methylcyclohexan.

Bevorzugt sind 1,6-Diisocyanatohexan, 1-Isocyanato-3,3,5- trimethyl-5-(isocyanatomethyl)cyclohexan, 4,4'-Di(isocyanatocyclohexyl)methan und Toluylendiisocyanat-Isomerengemische. Besonders bevorzugt sind 1,6-Diisocyanatohexan, 1-Isocyanato-3,3,5- trimethyl-5-(isocyanatomethyl)cyclohexan und 4,4'-Di(isocyanatocyclohexyl)methan.

Außer Diisocyanaten können mit dem erfindungsgemäßen Verfahren prinzipiell auch Monoisocyanate mit einer Isocyanatgruppe oder höhere Isocyanate mit im Mittel mehr als 2 Isocyanatgruppen. Dafür eignen sich beispielsweise Triisocyanate wie Triisocyanatononan, 2,4,6-Triisocyanatotoluol, Triphenylmethantriisocyanat oder 2,4,4'-Triisocyanatodiphenylether oder die Gemische aus Di-, Tri- und höheren Polyisocyanaten, die beispielsweise durch Phosgenierung von entsprechenden Anilin/Formaldehyd-Kondensaten erhalten werden und Methylenbrücken aufweisende Polyphenylpolyisocyanate darstellen.

Ein bevorzugtes Monoisocyanat stellt Phenylisocyanat dar.

Bevorzugt ist jedoch die Herstellung von Diisocyanaten.

Für das erfindungsgemäße Verfahren können solche Amine zur Reaktion zu den korrespondierenden Diisocyanaten eingesetzt werden, die bevorzugt ohne wesentliche Zersetzung, d.h. zu mindestens 95 Gew% unzersetzt, bevorzugt zu mindestens 98, besonders bevorzugt zu mindestens 99, ganz besonders bevorzugt zu mindestens 99,5, insbesondere zu mindestens 99,8, speziell zu mindestens 99,9 und sogar zu mindestens 99,95 Gew% unzersetzt, in die Gasphase überführt werden können. Besonders geeignet sind hier Amine, insbesondere Diamine, auf Basis von aliphatischen oder cycloaliphatischen Kohlenwasserstoffen mit 2 bis 18 Kohlenstoffatomen. Beispiele hierfür sind 1,6-Diaminohexan, 1-Amino-3,3,5-trimethyl-5-aminomethylcyclohexan (IP-DA) und 4,4'-Diaminodicyclohexylmethan. Bevorzugt verwendet wird 1,6-Diaminohexan (HDA).

Ebenfalls können für das erfindungsgemäße Verfahren aromatische Amine verwendet werden, die bevorzugt ohne wesentliche Zersetzung in die Gasphase überführt werden können. Beispiele für bevorzugte aromatische Amine sind Toluylendiamin (TDA), als 2,4- oder 2,6-Isomer oder als Gemisch davon, Diaminobenzol, R,S-1-Phenylethylamin, 1-Methyl-3-phenylpropylamin, 2,6-Xylidin, 3,3'-Diaminodiphenylsulfon, Napthyldiamin (NDA) und 2,4'- oder 4,4'-Methylen(diphenylamin) (MDA) oder Isomerengemische davon. Bevorzugt sind unter diesen die Diamine, besonders bevorzugt ist 2,4- und/oder 2,6-TDA.

Die Edukte oder auch nur eines von ihnen können, gegebenenfalls einzeln oder jeweils zusammen mit einem Inertmedium in den Reaktionsraum eindosiert werden.

Im erfindungsgemäßen Verfahren kann dem Phosgenstrom und/oder Aminstrom ein zusätzliches Inertmedium beigemischt werden. Bei dem Inertmedium handelt es sich um ein Medium, das bei der Reaktionstemperatur gasförmig im Reaktionsraum vorliegt und im wesentlichen nicht mit den im Reaktionsverlauf auftretenden Verbindungen reagiert bzw. unter den Reaktionsbedingungen stabil ist. Bevorzugt sind solche Inertmedien, die unter den Reaktionsbedingungen zu mindestens 95 Gew% unzersetzt und unreagiert bleiben, bevorzugt zu mindestens 98, besonders bevorzugt zu mindestens 99, ganz besonders bevorzugt zu mindestens 99,5, insbesondere zu mindestens 99,8, speziell zu mindestens 99,9 und sogar zu mindestens 99,95 Gew%. Das Inertmedium wird im allgemeinen vor der Umsetzung mit Amin und/oder Phosgen vermischt, kann aber auch getrennt von den Eduktströmen beispielsweise direkt in die Reaktionszone dosiert werden. Beispielsweise können Stickstoff, Kohlenstoffdioxid oder Kohlenstoffmonoxid, Edelgase, wie Helium oder Argon, oder Aromaten, wie Toluol oder Xylol oder chlorierte Aromaten, wie Chlorbenzol oder Dichlorbenzol, verwendet werden. Bevorzugt wird Stickstoff und/oder Chlorbenzol als Inertmedium verwendet.

Im allgemeinen wird das Inertmedium in einer Menge eingesetzt, so dass das Verhältnis der Gasvolumina von Inertmedium zu Amin bzw. zu Phosgen mehr als 0,0001 bis 30, bevorzugt mehr als 0,001 bis 15, besonders bevorzugt mehr als 0,001 bis 5 beträgt.

Bevorzugt wird das Inertmedium zusammen mit dem Diamin in den Reaktionsraum eingeführt. Es sind jedoch auch andere Dosierungsmöglichkeiten denkbar.

Die Zuführung von Phosgen über den phosgenhaltigen Strom zum Reaktionsraum kann auch so erfolgen, dass man statt eines einzelnen phosgenhaltigen Stroms mehrere phosgenhaltige Teilströme zuführt. In einem solchen Fall werden die phosgenhaltigen Teilströme zu einem gesamten phosgenhaltigen Gesamtstrom addiert.

Derartige Teilströme können in folgender Weise dosiert werden:
- Man kann verschiedene phosgenhaltige Teilströme, beispielsweise rückgeführtes Phosgen und Frisch-Phosgen, vor der Einspeisung zu einem phosgenhaltigen Gesamtstrom vereinigen und in den Reaktionsraum einspeisen.
- Man kann mehrere Teilströme, wobei es sich jeweils um rückgeführtes Phosgen, Frisch-Phosgen oder Gemische daraus handeln kann, an der gleichen Stelle in den Reaktionsraum einspeisen. Dies kann beispielsweise über mehrere Düsen erfolgen, die parallel um eine Zentraldüse angeordnet sind, wie es beispielsweise in EP 1449826 A1 beschrieben ist, oder durch mehrfache Eindüsung in einen Ringraum zur Vermischung, bevor dieser Strom mit einem über eine Zentraldüse dosierten aminhaltigen Strom vermischt wird.
- Man kann mehrere Teilströme, wobei es sich jeweils um rückgeführtes Phosgen, Frisch-Phosgen oder Gemische daraus handeln kann, an verschiedenen Stellen des Reaktionsraums eindosieren, so daß Phosgen im Verlauf der Reaktion nachdosiert wird.

Mit dem Begriff "Frisch-Phosgen" ist ein phosgenhaltiger Strom bezeichnet, der nicht aus einem Phosgenierungsverfahren zurückgeführt worden ist, sondern der nach der Synthese des Phosgen, zumeist aus Chlor und Kohlenmonoxid, keine Reaktionstufe mit einer Phosgenumsetzung durchlaufen hat, in der mehr als 5 % Umsatz des in der Phosgensynthese hergestellten Phosgen erfolgt.

Es stellt eine bevorzugte Ausführungsform der vorliegenden Erfindung dar, daß das Frisch-Phosgen nach seiner Synthese und gegebenenfalls Aufreinigung in einer Anlage zur Herstellung von Phosgen (II) im wesentlichen gasförmig in das erfindungsgemäße Verfahren geführt wird. Dazu ist das erfindungsgemäße Verfahren bevorzugt direkt, besonders bevorzugt über eine Rohrleitung, mit einer Anlage zur Herstellung von Phosgen verbunden.

Dies hat zur Folge, daß einerseits erfindungsgemäß auf die Zuführung von Energie zur Verdampfung von flüssigem Phosgen verzichtet werden kann und andererseits die Bevorratung an Phosgen auf das in dieser Rohrleitung befindliche gasförmige Phosgen begrenzt ist.

Häufig wird Phosgen durch Kontaktieren von Kohlenstoffmonoxid mit molekularem Chlor (Cl₂) an geeigneten Trägern, bevorzugt Aktivkohle, hergestellt. Da die Bildung von Phosgen stark exotherm ist, erfolgt diese bevorzugt in Rohrbündelreaktoren, in denen die Trägerschüttung durch die exotherme Reaktion auf Temperaturen bis zu 400 °C erhitzt wird, wobei die Temperatur jedoch beim Durchgang durch die Rohre auf 40 bis 150 °C absinkt. Die freigesetzte Reaktionswärme wird dabei durch geeignete Wärmeträgermedien, beispielsweise Monochlorbenzol, Dichlorbenzol oder Wasser, abgeführt. Denkbar sind auch Wärmeträgeröle. Dabei liegt zumeist normaler Druck oder leichter Überdruck an.

Das zu mindestens 75 Gew% gasförmig, bevorzugt zu mindestens 85, besonders bevorzugt zu mindestens 90, ganz besonders bevorzugt zu mindestens 95, insbesondere zu mindestens 98, speziell zu mindestens 99 und sogar zu mindestens 99,5 Gew% gasförmig erhaltene Phosgen kann bevorzugt ohne weitere Aufreinigung und insbesondere ohne Zwischenkondensation in die Gasphasenphosgenierung eingesetzt werden. Falls gewünscht können beispielsweise durch eine Wäsche Nebenprodukte durch Absorption aus dem Reaktionsgemisch entfernt werden.

Es kann aber auch sinnvoll sein, das eingesetzte Amin als Aerosol einzusetzen, d.h. im wesentlichen als fein verteilte Flüssigkeit mit einer Tropfengrößenverteilung von 10 nm bis 1 mm, vorzugsweise von 100 nm bis 100 µm insbesondere von 0,2 bis 10 µm aufweisen. Die Tropfengrößenverteilung kann sehr breit oder sehr eng zwischen diesen Grenzen sein. Im idealen Fall ist die Tropfengrößenverteilung sehr eng. Als Maß für die Breite der Verteilung dient die auf den d50 der Tropfengrößenverteilung normierte Standardabweichung σ. Der d50 stellt die Tropfengröße dar, für die die Summenverteilungsfunktion den Wert 0,5 (50 %) erreicht. Für eine sehr breite Verteilung ist σ >> 1. Für eine enge Verteilung gilt σ < 1 und für eine ideal monodisperse Verteilung der Wert σ = 0.

Allgemein gilt, dass die Größe der Tröpfchen so klein wie möglich sein sollte, da dies eine hohe Eindringgeschwindigkeit des Phosgens in die flüssige aminhaltige Phase gewährleistet. Desweiteren wird durch den realisierbaren Tropfendurchmesser die maximale Größe der ausfallenden Aminhydrochloridpartikel beschränkt. Deshalb gilt auch hier, dass ein feines gegenüber einem sehr groben Aerosol vorzuziehen ist. Allerdings muss darauf geachtet werden, dass das erzeugte Aerosol/Produkt auch von den nachgeschalteten Tropfen-/Staubabscheidern abgeschieden wird.

Ein solches Verfahren ist beispielsweise beschrieben in der Internationalen Patentanmeldung WO 2008/006775, auf die hiermit in vollem Umfang Bezug genommen sei. ,

Es stellt eine bevorzugte Ausführungsform der vorliegenden Erfindung dar, daß die Herstellung von Phosgen mit einem stöchiometrischen Überschuß von Kohlenstoffmonoxid erfolgt, wobei in der Regel eine vollständige Umsetzung des Chlor erreicht wird. Das auf diese Wiese erhaltene Phosgen enthält noch geringe Mengen, in der Regel nicht mehr als 5 Gew% Kohlenstoffmonoxid. Dies hat auf die Anwendung in dem erfindungsgemäßen Verfahren jedoch keine nachteilige Auswirkung, da das enthaltene Kohlenmonoxid als Inertgas in der Gasphasenphosgenierung fungiert. Das enthaltene Kohlenmonoxid wird dann in der Regel in der Chlorwasserstoffabtrennung (IX) (siehe unten) ausgeschleust.

Es stellt eine bevorzugte Ausführungsform dar, das Phosgen nicht direkt in die Synthese sondern zunächst in die Phosgenreinigung in Stufe (IX) (siehe unten) zu führen.

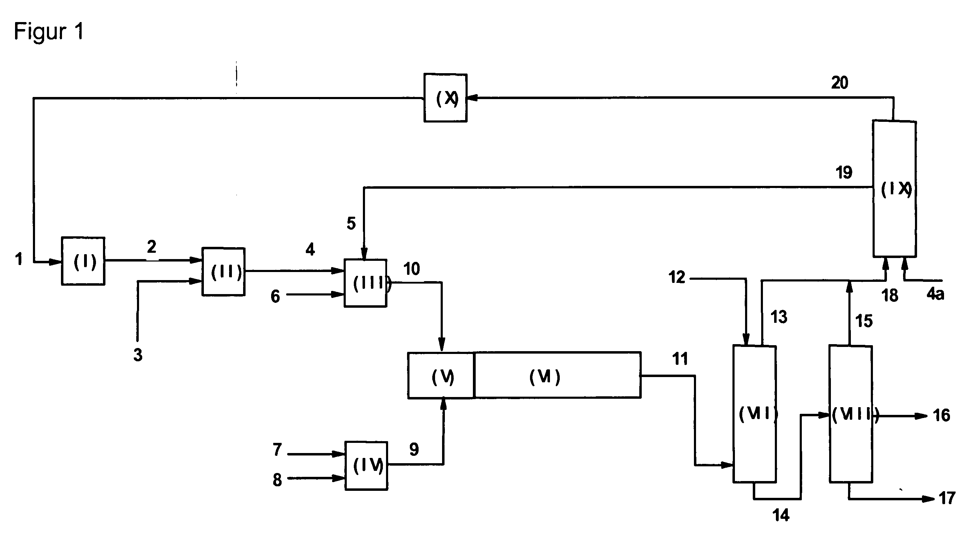

Diese Ausführungsform ist durch Strom 4a in Figur 1 dargestellt.

Dies kann insbesondere sinnvoll sein, um dort Spuren von molekularem Chlor abzutrennen, so daß diese Ausführungsform insbesondere dann bevorzugt ist, wenn das aus der Phosgensynthese erhaltene Phosgen noch Spuren von molekularem Chlor (Cl₂) enthält, beispielsweise bei einem Gehalt von mehr als 5 ppm, bevorzugt bei mehr als 3 ppm, besonders bevorzugt bei mehr als 1 ppm, ganz besonders bevorzugt bei mehr als 0,5 ppm und insbesondere bei einem Gehalt von mehr als 0,1 ppm.

Zur Durchführung des erfindungsgemäßen Verfahrens kann es vorteilhaft sein, die Ströme der Edukte Amin und/oder Phosgen vor dem Vermischen vorzuwärmen, bevorzugt zu überhitzen, üblicherweise auf Temperaturen von mindestens 200 °C, bevorzugt mindestens 260 °C und besonders bevorzugt mindestens 300 °C.

In der Aminvorlage (IV) wird das Amin bevorzugt zusammen mit einem Inertmedium als Trägergas wie oben beschrieben in die Gasphase überführt und in die Mischeinheit (V) eingespeist. Das Amin kann aber auch direkt ohne Verwendung eines Inertmediums verdampft und in die Mischeinheit (V) geführt werden.

Der gasförmige Phosgenstrom, enthatend frisches und rückgeführtes Phosgen, wird auf die genannte Temperatur überhitzt. Gegebenenfalls kann er mit mindestens einem Inertmedium vermischt und anschließend der Mischeinheit (V) zugeführt werden.

Im erfindungsgemäßen Verfahren erfolgt die Vermischung der Reaktanden in einer Mischeinrichtung (V), die sich durch eine hohe Scherung der durch die Mischeinrichtung geführten Reaktionsströme auszeichnet. Bevorzugt werden als Mischeinrichtung eine statische Mischeinrichtung oder eine Mischdüse verwendet, die der Reaktionszone (VI) vorangestellt ist. Besonders bevorzugt wird eine Mischdüse verwendet.

Die Art der Mischung spielt erfindungsgemäß keine Rolle und kann auf beliebige Art und Weise erfolgen, beispielsweise wie beschrieben in EP-B1 699657, EP-A2 1319655, Sp. 1, Z. 54 bis Sp. 2, Z. 24 und Sp. 4, Z. 16 - 40, EP-A1 1275640, Sp. 3, Z. 27 - Sp. 4, Z. 5, EP-A2 1362847, Sp. 2, Z. 19 - Sp. 3, Z. 51 und Sp. 4, Z. 40 - Sp. 5, Z. 12, auf die jeweils im Umfang dieser Offenbarung ausdrücklich Bezug genommen sei.

Im Verfahren wird typischerweise Phosgen im Überschuss bezüglich Aminogruppen eingesetzt. Üblicherweise liegt ein molares Verhältnis von Phosgen zu Aminogruppen von 1,1 :1 bis 20 : 1, bevorzugt von 1,2 :1 bis 5 :1 vor.

Nach dem Vermischen in der Mischeinheit (V) wird das gasförmige Gemisch aus Phosgen, Amin und gegebenenfalls Inertmedium in den Reaktor geführt, wobei der Reaktor den Reaktionsraum enthält:

Die Umsetzung von Phosgen mit Amin erfolgt in einem Reaktionsraum, der im allgemeinen in einem Reaktor angeordnet ist, d.h. unter Reaktionsraum (VI) wird der Raum verstanden, in dem ein für die Ausbeute des Verfahrens relevanter Teil der Umsetzung der Edukte und Zwischenprodukte und/oder der Produkte erfolgt, in dem beispielsweise mindestens 0,5 mol% des eingesetzten Amins verbraucht und/oder des korrespondierenden Isocyanats gebildet wird, bevorzugt mindestens 1 mol%, besonders bevorzugt mindestens 3 mol%, ganz besonders bevorzugt mindestens 5 mol%, insbesondere mindestens 7 mol% und speziell mindestens 10 mol% des eingesetzten Amins verbraucht und/oder des korrespondierenden Isocyanats gebildet wird.

Unter Reaktor wird die technische Vorrichtung verstanden, die den Reaktionsraum enthält. Hierbei kann es sich um alle üblichen, aus dem Stand der Technik bekannten Reaktionsräume handeln, die zur nicht katalytischen, einphasigen Gasreaktion, bevorzugt zur kontinuierlichen nicht katalytischen, einphasigen Gasreaktion, geeignet sind und die den geforderten Drücken standhalten. Geeignete Materialien für den Kontakt mit dem Reaktionsgemisch sind z. B. Metalle, wie Stahl, insbesondere legierter Stahl, Tantal, Nickel, Nickellegierungen, Silber oder Kupfer, Glas, Keramik, Emaille oder homogene oder heterogene Gemische und Bauteile daraus. Bevorzugt werden Stahlreaktoren verwendet. Die Wände des Reaktors können glatt oder profiliert sein. Als Profile eignen sich beispielsweise Ritzen oder Wellen.

Es können im allgemeinen die aus dem Stand der Technik bekannten Reaktorbautypen verwendet werden. Beispiele für Reaktoren sind bekannt aus EP-B1 289840, Sp. 3, Z. 49 - Sp. 4, Z. 25, EP-B1 593334, WO 2004/026813, S. 3, Z. 24 - S. 6, Z. 10, WO 03/045900, S. 3, Z. 34 - S. 6, Z. 15, EP-A1 1275639, Sp. 4, Z. 17 - Sp. 5, Z. 17 und EP-B1 570799, Sp. 2, Z. 1 - Sp. 3, Z. 42, auf die jeweils im Umfang dieser Offenbarung ausdrücklich Bezug genommen sei.

Bevorzugt verwendet werden Rohrreaktoren.

Ebenfalls ist es möglich im wesentlichen quaderförmige Reaktionsräume, bevorzugt Plattenreaktoren bzw. Plattenreaktionsräume zu verwenden. Ein besonders bevorzugter Plattenreaktor weist ein Verhältnis von Breite zu Höhe von mindestens 2 : 1, bevorzugt mindestens 3 : 1, besonders bevorzugt mindestens 5 : 1 und insbesondere mindestens 10 : 1 auf. Die obere Grenze des Verhältnisses von Breite zu Höhe hängt von der gewünschten Kapazität des Reaktionsraums ab und ist prinzipiell nicht begrenzt. Technisch sinnvoll haben sich Reaktionsräume mit einem Verhältnis von Breite zu Höhe bis maximal 5000 : 1, bevorzugt 1000 : 1 erwiesen.

Die Umsetzung von Phosgen mit Amin im Reaktionsraum (VI) erfolgt bei Absolutdrücken von mehr als 0,1 bar bis weniger als 20 bar, bevorzugt zwischen 0,5 bar und 10 bar, besonders bevorzugt zwischen 0,7 bar und 5 bar, insbesondere von 0,8 bis 3 bar.

Im allgemeinen ist der Druck in den Zuleitungen zur Mischvorrichtung höher, als der vorstehend angegebene Druck im Reaktor. Je nach Wahl der Mischvorrichtung fällt an dieser Druck ab. Bevorzugt ist der Druck in den Zuleitungen um 20 bis 10000 mbar, besonders bevorzugt von 30 bis 7000 mbar höher als im Reaktionsraum.

In einer bevorzugten Ausführungsform besteht der Reaktor aus einem Bündel von Reaktoren. In einer möglichen Ausführungsform muss es sich bei der Mischeinheit nicht um eine eigenständige Vorrichtung handeln, vielmehr kann es vorteilhaft sein, die Mischeinheit in den Reaktor zu integrieren. Ein Beispiel einer integrierten Einheit aus Mischeinheit und Reaktor stellt ein Rohrreaktor mit angeflanschten Düsen dar.

Im allgemeinen ist der Druck in der Aufarbeitungsvorrichtung niedriger als im Reaktionsraum.

Gemäß dem erfindungsgemäßen Verfahren erfolgt die Umsetzung von Phosgen mit Amin in der Gasphase. Unter Umsetzung in der Gasphase ist zu verstehen, dass die Umwandlung der Eduktströme und Zwischenprodukte zu den Produkten im gasförmigen Zustand miteinander reagieren und im Verlauf der Reaktion während des Durchgangs durch den Reaktionsraum zu mindestens 95%, bevorzugt zu mindestens 98%, besonders bevorzugt zu mindestens 99%, ganz besonders bevorzugt zu mindestens 99,5%, insbesondere zu mindestens 99,8 und speziell zu mindestens 99,9% in der Gasphase bleiben.

Zwischenprodukte sind dabei beispielsweise die aus den Diaminen gebildeten Monoamino-monocarbamoylchloride, Dicarbamoylchloride, Monoamino-monoisocyanate und Monoisocyanato-monocarbamoylchloride sowie die Hydrochloride der Aminoverbindungen.

Bei dem erfindungsgemäßen Verfahren wird die Temperatur im Reaktionsraum (VI) so gewählt, dass sie oberhalb der Dissoziationstemperatur der Hydrochloride des eingesetzten Diamins, bezogen auf die im Reaktionsraum herrschenden Partialdruckverhältnisse, liegt. Je nach eingesetztem Amin und eingestelltem Druck ergibt sich üblicherweise eine vorteilhafte Temperatur im Reaktionsraum von mehr als 200 °C, bevorzugt mehr als 260 °C und besonders bevorzugt mehr als 300 °C. In der Regel beträgt die Temperatur bis zu 600, bevorzugt bis zu 570 °C.

Die mittlere Kontaktzeit des Umsetzungsgemisches im erfindungsgemäßen Verfahren beträgt im allgemeinen zwischen 0,001 Sekunden und weniger als 5 Sekunden, bevorzugt von mehr als 0,01 Sekunden bis weniger als 3 Sekunden, besonders bevorzugt von mehr als 0,02 Sekunden bis weniger als 1,5 Sekunden. Unter mittlerer Kontaktzeit wird die Zeitspanne vom Beginn der Vermischung der Edukte bis zum Verlassen des Reaktionsraumes in die Aufarbeitungsstufe verstanden. In einer bevorzugten Ausführungsform ist die Strömung im erfindungsgemäßen Verfahren durch eine Bodenstein-Zahl von mehr als 10, bevorzugt mehr als 100 und besonders bevorzugt von mehr als 500 charakterisiert.

In einer bevorzugten Ausführungsform werden die Abmessungen des Reaktionsraums und die Strömungsgeschwindigkeiten so gewählt, dass eine turbulente Strömung, d.h. eine Strömung mit einer Reynolds-Zahl von mindestens 2300, bevorzugt mindestens 2700, für das Reaktionsgemisch vorliegt, wobei die Reynolds-Zahl mit dem hydraulischen Durchmesser des Reaktionsraumes gebildet wird.

Bevorzugt durchlaufen die gasförmigen Reaktionspartner den Reaktionsraum mit einer Strömungsgeschwindigkeit von 3 bis 400 Meter/Sekunde, bevorzugt von 10 bis 250 Meter/Sekunde. Durch die turbulente Strömung werden eine enge Verweilzeit mit geringer Standardabweichung von meist nicht mehr als 6% wie in EP 570799 beschrieben und eine gute Vermischung erreicht. Maßnahmen, wie beispielsweise die in EP-A-593 334 beschriebene Verengung, die zudem verstopfungsanfällig ist, sind nicht notwendig.

Das Reaktionsvolumen kann über seine Außenfläche temperiert werden. Um Produktionsanlagen mit hoher Anlagenkapazität zu bauen, können mehrere Reaktorrohre parallel geschalten werden. Die Umsetzung kann aber auch adiabat erfolgen. Das bedeutet, dass über die Außenfläche des Reaktionsvolumens nicht mit technischen Maßnahmen Heiz- oder Kühlenergieströme fließen. Bevorzugt findet die Umsetzung adiabat statt.

Das Phosgenierungsverfahren wird bevorzugt einstufig durchgeführt. Darunter ist zu verstehen, dass die Vermischung (V) und Umsetzung (VI) der Edukte in einem Schritt und in einem Temperaturbereich, bevorzugt in dem vorstehend genannten Temperaturbereich, erfolgt. Ferner wird das erfindungsgemäße Verfahren bevorzugt kontinuierlich durchgeführt.

Nach der Reaktion führt man das Umsetzungsgemisch einer Mischeinrichtung VII (Quench) zu, in der man die Temperatur des Gases durch Einbringen einer kühleren Flüssigkeit absenkt. Ausführungsformen dieser Verfahrensstufe können sein: Waschtürme, Rührbehälter, Blasensäulen, Quenchdüsen und ähnliches. In ihnen wird durch Kontakt mit mindestens einem, bevorzugt genau einem inerten Lösungsmittel das gebildete Isocyanat aus dem gasförmigen Reaktionsgemisch auskondensiert, während überschüssiges Phosgen, Chlorwasserstoff und gegebenenfalls das Inertmedium diese Aufarbeitungsvorrichtung im wesentlichen gasförmig durchlaufen.

Als inertes Lösungsmittel sind bevorzugt Kohlenwasserstoffe, die gegebenenfalls mit Halogenatomen substituiert sind, geeignet, wie beispielsweise Chlorbenzol, Dichlorbenzol, 1,3,5-Trimethylbenzol (Mesitylen), Xylol und Toluol. Bevorzugt wird dabei die Temperatur des inerten Lösungsmittels oberhalb der Schmelztemperatur des zum Amin gehörigen Carbamylchlorids gehalten. Besonders bevorzugt wird dabei die Temperatur des inerten Lösungsmittels oberhalb der Lösungstemperatur des zum Amin gehörigen Carbamylchlorids im gewählten Quenchmedium gehalten.

Verfahrenstechnisch können für diese Verfahrensstufe im erfindungsgemäßen Verfahren alle an sich bekannten Extraktions- und Waschverfahren und -apparate eingesetzt werden, z.B. solche, die in Ullmann's Encyclopedia of Industrial Chemistry, 6th ed, 1999 Electronic Release, Kapitel: Liquid - Liquid Extraction - Apparatus, beschrieben sind. Beispielsweise können dies ein- oder mehrstufige, bevorzugt einstufige Extraktionen, sowie solche in Gleich- oder Gegenstromfahrweise, bevorzugt Gegenstromfahrweise sein.

Ein weiterer geeigneter Quench ist beispielsweise bekannt aus EP-A1 1403248, Sp. 2, Z. 39 - Sp. 3, Z. 18, auf die im Umfang dieser Offenbarung ausdrücklich Bezug genommen sei. Des weiteren kann der Quench auch ausgeführt sein, wie beschrieben in WO 2008/055899 und WO 2008/055904 oder wie in der internationalen Anmeldung WO 2005/123665.

In der Verfahrensstufe (VII) wird das Reaktionsgemisch, das im wesentlichen aus den Isocyanaten, Phosgen und Chlorwasserstoff besteht, intensiv mit der eingedüsten Flüssigkeit vermischt. Die Vermischung erfolgt derart, dass die Temperatur des Reaktionsgemisches ausgehend von 200 bis 570°C auf 100 bis 200 °C, bevorzugt auf 100 bis 180 °C abgesenkt wird und das im Reaktionsgemisch enthaltene Isocyanat durch Kondensation vollständig oder teilweise in die eingedüsten Flüssigkeitströpfchen übergeht, während das Phosgen und der Chlorwasserstoff im wesentlichen vollständig in der Gasphase verbleiben.

Der Anteil des im gasförmigen Reaktionsgemisch enthaltenen Isocyanats, das in der Quenchzone in die Flüssigphase übergeht, beträgt dabei vorzugsweise 20 bis 100 Gew.-%, besonders bevorzugt 50 bis 99,5 Gew.-% und insbesondere 70 bis 99 Gew.-%, bezogen auf das gesamte im Reaktionsgemisch enthaltene Isocyanat.

Der Anteil des im gasförmigen Reaktionsgemisch enthaltenen Phosgens und Chlorwasserstoffs, das in der Quenchzone in die Flüssigphase übergeht, beträgt dabei in der Regel weniger als 25 Gew.-%.

Im Reaktionsgemisch sollte der Umsatz an Aminogruppen mindestens 95 %, bevorzugt mindestens 98, besonders bevorzugt mindestens 99, ganz besonders bevorzugt mindestens 99,5, insbesondere mindestens 99,8 und speziell mindestens 99,9 % betragen.

Das Reaktionsgemisch durchströmt die Quenchzone vorzugsweise von oben nach unten. Unterhalb der Quenchzone ist ein Sammelbehälter angeordnet, in dem die Flüssigphase abgeschieden, gesammelt und, über einen Auslass entfernt und anschließend aufgearbeitet wird. Die verbleibende Gasphase wird über einen zweiten Auslass aus dem Sammelbehälter entfernt und ebenfalls aufgearbeitet.

Die Quenchflüssigkeit muss eine gute Löslichkeit für Isocyanate aufweisen. Vorzugsweise werden organische Lösungsmittel eingesetzt. Insbesondere eingesetzt werden aromatische Lösungsmittel, die mit Halogenatomen substituiert sein können. Beispiele für derartige Flüssigkeiten sind Toluol, Benzol, Nitrobenzol, Anisol, Chlorbenzol, Dichlorbenzol (ortho oder para oder Isomerengemische daraus), Trichlorbenzol, 1,3,5-Trimethylbenzol (Mesitylen), Xylol, Hexan, Diethylisophthalat (DEIP), Tetrahydrofuran (THF), Dimethylformamid (DMF) und deren Gemische, bevorzugt Monochlorbenzol.

In einer besonderen Ausführungsform des erfindungsgemäßen Verfahrens handelt es sich bei der eingedüsten Flüssigkeit um ein Gemisch aus Isocyanaten, ein Gemisch aus Isocyanaten und Lösungsmittel oder um Isocyanat, wobei die jeweils verwendete Quenchflüssigkeit Anteile an Leichtsieder, wie Chlorwasserstoff und Phosgen, aufweisen kann. Vorzugsweise wird dabei das Isocyanat eingesetzt, das bei dem jeweiligen Verfahren hergestellt wird. Da durch die Temperatursenkung in der Quenchzone die Reaktion zum Stillstand kommt, können Nebenreaktionen mit den eingedüsten Isocyanaten ausgeschlossen werden. Der Vorteil dieser Ausführungsform liegt insbesondere darin, dass auf eine Abtrennung des Lösungsmittels verzichtet werden kann.

In einer alternativen bevorzugten Ausführungsform handelt es sich bei dem Inertmedium, das zusammen mit mindestens einem der Edukte eingesetzt wird, und bei dem Lösungsmittel, das im Quench eingesetzt wird, um die gleiche Verbindung, ganz besonders bevorzugt wird in diesem Fall Monochlorbenzol verwendet.

Die aus der Stufe (VII) ausgetragene Quenchflüssigkeit kann neben Diisocyanat auch bis zu 10 Gew% an Phosgen und/oder bis zu 10 Gew% Chlorwasserstoff enthalten.

Um die Absorption von Phosgen in der Quenchflüssigkeit erfindungsgemäß niedrig zu halten wird der Quench bevorzugt bei niedrigem Druck, beispielsweise 1 bis 5 bar, und erhöhter Temperatur, beispielsweise 100 bis 160 °C, durchgeführt.

Geringe Mengen von Nebenprodukten, die im Isocyanat verbleiben, können mittels zusätzlicher Rektifikation, durch Strippen mit einem Inertgas oder auch Kristallisation, bevorzugt durch Rektifikation vom erwünschten Isocyanat getrennt werden.

In der anschließenden optionalen Reinigungsstufe (VIII) wird das Isocyanat, bevorzugt durch Destillation oder besonders bevorzugt durch Rektifikation, vom Lösungsmittel abgetrennt. Ebenfalls kann hier noch die Abtrennung von restlichen Verunreinigungen, umfassend Chlorwasserstoff, Inertmedium und/oder Phosgen, erfolgen, wie beispielsweise beschrieben in DE-A1 10260092.

Aus dem Quench (VII) und/oder der Reinigungsstufe (VIII) werden gasförmige Stoffströme, die im wesentlichen aus Phosgen und/oder Chlorwasserstoffgas und/oder Inertmedium bestehen, erhalten. Aus zumindest einem Teil dieser Phosgen und/oder Chlorwasserstoffgas und/oder Inertmedium enthaltenden Stoffströme wird in Stufe (IX) Chlorwasserstoff vom Phosgen abgetrennt.

Dies kann beispielsweise erfolgen, wie es in der Internationalen Anmeldung WO 2007/014936 beschrieben ist. Hier wird in einer Wäsche Chlorwasserstoff aus dem Gasgemisch in einer geeigneten Waschflüssigkeit absorbiert. Das verbleibende Phosgen verlässt die Kolonne gasförmig und kann in die Reaktion zurückgeführt werden. Als geeignete Waschflüssigkeiten sind in WO 2007/014936 ionische Flüssigkeiten beschrieben. Diese Verfahrensvariante ist besonders vorteilhaft, da sie Phosgen in kondensierter Phase vermeidet.

In einer weiteren bevorzugten Ausführungsform erfolgt die Abtrennung so, daß der Massenbruch von Chlorwasserstoff im phosgenhaltigen Strom nach gegebenenfalls durchgeführter Vermischung mit Frisch-Phosgen vor der Vermischung mit dem aminhaltigen Strom weniger als 15 Gew.-%, bevorzugt weniger als 10, besonders bevorzugt weniger als 5 Gew% beträgt.

Die Trennung des Chlorwasserstoff und/oder Phosgen sowie gegebenenfalls Lösungsmittel aus dem Quench enthaltenden Gemisches erfolgt bevorzugt über Destillation und/oder eine Wäsche. Bevorzugt wird die Trennung in einer Kombination von einer Destillation und einer Wäsche durchgeführt.

Bevorzugte Waschmedien für die Phosgen-Chlorwasserstofftrennung (IX) sind die oben als Quenchmedien aufgeführten Lösungsmittel. Besonders bevorzugt werden als Wasch- und als Quenchmedium in den Stufen (VII) und (IX) die gleichen Lösungsmittel eingesetzt.

Bei einer kombinierten Wäsche und Destillation, beispielsweise einer Druckdestillation, wird Phosgen aus dem chlorwasserstoffhaltigen Strom durch Wäsche mit einem Waschmedium, bevorzugt Toluol, Chlorbenzol oder Dichlorbenzol, ausgewaschen. Hier liegt ausnahmsweise im erfindungsgemäßen Verfahren Phosgen in kondensierter Form vor. Die Abtrennung von Phosgen und Chlorwasserstoff aus dem beladenen Waschmedium nach der Wäsche erfolgt bevorzugt destillativ oder durch Desorption. Dabei erfolgt die Abtrennung bevorzugt so, dass ein gasförmiger Phosgenstrom anfällt, der in einer besonders bevorzugten Ausführungsform, gegebenenfalls nach Vermischung mit Frischphosgen, einen Gehalt an Chlorwasserstoff von kleiner 15 Gew% enthält.

Die Wäsche und die Destillation wird bei Drücken von 1 bis 10 bar absolut betrieben, bevorzugt von 1 bis 5 bar absolut.

Der Chlorwasserstoff-/Phosgentrennung (IX) kann eine Adsorbtionseinheit, bevorzugt ein Aktivkohlefilter, im aus der Trennung abgeführten Chlorwasserstoffsstrom nachgeschalten werden, in dem Spuren des Waschmediums aus dem anfallenden Chlorwasserstoff entfernt werden.

Eine bevorzugte Ausführungsform des erfindungsgemäßen Verfahrens ist in Figur 1 schematisch veranschaulicht.

In Figur 1 bedeutet:
- I: Herstellungseinheit für Chlor (Cl₂)
- II: Herstellungseinheit für Phosgen
- III: Vermischung Phosgen und Inertgas
- IV: Amin-Vorlage und optionale Vermischung mit Inertgas
- V: Mischeinheit für phosgenhaltigen und aminhaltigen Strom
- VI: Reaktionsraum
- VII: Aufarbeitungsstufe (Quench)
- VIII: Reinigungsstufe
- IX: Phosgen-Chlorwasserstoff-Trennung
- X: Absorption zur Abtrennung von Waschmedium (optional)

- 1: Zufuhr Chlorwasserstoff (HCl)
- 2: Zufuhr Chlor (Cl₂)
- 3: Zufuhr Kohlenstoffmonoxid
- 4: Zufuhr Phosgen (frisch)
- 5: Zufuhr Phosgen (rückgeführt)
- 6: Zufuhr Inertmedium
- 7: Zufuhr Amin
- 8: Zufuhr Inertmedium
- 9: aminhaltiger Strom
- 10: phosgenhaltiger Strom
- 11: Reaktionsgemisch
- 12: Quenchflüssigkeit
- 13: gasförmiger Kopfaustrag Quench
- 14: beladene Waschflüssigkeit
- 15: gasförmiger Brüden
- 16: Waschflüssigkeit
- 17: Isocyanat (roh)
- 18: vereinigte gasförmige Ströme
- 19: Phosgen (rückgeführt)
- 20: Chlorwasserstoffrückführung

In der Amin-Vorlage (IV) wird das Amin 7, gegebenenfalls zusammen mit einem Inertmedium 8 als Trägergas, wie beispielsweise Stickstoff, in die Gasphase überführt und in die Mischeinheit (V) eingespeist. Ebenfalls wird gasförmiges Phosgen 4 direkt oder indirekt aus der Phosgenproduktion (II) erhalten, optional in Stufe (III) mit einem Inertmedium 6 vermischt und in die Mischeinheit (V) geführt. Nach dem Vermischen in der Mischeinheit (V), die beispielsweise aus einer Düse oder einem statischen Mischer bestehen kann, wird das gasförmige Gemisch aus Phosgen, Amin und gegebenenfalls Inertmedium in die Reaktionszone (VI) überführt. Wie in Figur 1 veranschaulicht muss es sich bei der Mischeinheit nicht um eine eigenständige Reaktionsstufe handeln, vielmehr kann es vorteilhaft sein, die Mischeinheit in die Reaktionszone (VI) zu integrieren.

Nach der Umsetzung in der Reaktionszone gelangt das Reaktionsgemisch 11 in die Aufarbeitungsstufe (VII). Bevorzugt handelt es sich hier um einen sogenannten Waschturm, wobei aus dem gasförmigen Gemisch das gebildete Isocyanat durch Kondensation in einem inerten Lösungsmittel 12 abgetrennt wird, während überschüssiges Phosgen, Chlorwasserstoff und gegebenenfalls das Inertmedium die Aufarbeitungsstufe gasförmig durchlaufen und am Kopf als Strom 13 abgenommen werden. Als inertes Lösungsmittel sind bevorzugt aromatische Kohlenwasserstoffe, die gegebenenfalls mit Halogenatomen substituiert sind, geeignet, wie beispielsweise Chlorbenzol oder Dichlorbenzol, Xylol und Toluol. Besonders bevorzugt wird dabei die Temperatur des inerten Lösungsmittels oberhalb der Lösungstemperatur des zum Amin korrespondierenden Carbamoylchlorids im gewählten Quenchmedium gehalten.

In der anschließenden Reinigungsstufe (VIII) wird das Isocyanat 17, bevorzugt durch Rektifikation, vom Lösungsmittel 16 abgetrennt. Ebenfalls kann hier noch die Abtrennung von restlichen Verunreinigungen, beispielsweise Chlorwasserstoff, Inertmedium und/oder Phosgen (Strom 15) erfolgen.

In der Regel wird das so erhaltene rohe Isocyanat 17 in einer anschließenden Reinigung (in Figur 1 nicht dargestellt), bevorzugt einer Destillation, noch weiter aufgereinigt, so daß die gewünschte Reinheit von beispielsweise mindestens 98 Gew%, bevorzugt mindestens 99, besonders bevorzugt mindestens 99,5 und ganz besonders bevorzugt mindestens 99,8 Gew% erhalten wird.

Das in der Reinigungsstufe (VIII) erhaltene Lösungsmittel 16 kann in der Regel ohne weitere Reinigung in die Aufarbeitungsstufe (VII) rückgeführt wird (Strom 12). Falls erforderlich kann dieser Teilstrom jedoch auch, bevorzugt destillativ, aufgereinigt werden.

Mindestens ein phosgenhaltiger Teilstrom 13 oder 15 der aus der Reinigungsstufe VIII und/oder der Aufarbeitungsstufe VII stammt, bevorzugt diese vereinigten Teilströme 18, werden in einer Stufe (IX) von darin enthaltenem Chlorwasserstoff zumindest teilweise befreit und Phosgen als Strom 19 in die Reaktion zurückgeführt. Dazu wird er mit dem Frischphosgenstrom 4 bzw. mit dem Phosgenstrom zur Mischeinrichtung (V) vereinigt oder getrennt in die Mischeinrichtung (V) geführt.

Bevorzugt werden Phosgen-Rückführstrom 19 und Frischphosgenstrom 4 in einem solchen Verhältnis miteinander vermischt, daß die in der Umsetzung angestrebte Stöchiometrie erreicht wird.

Die Entfernung des Chlorwasserstoffs in der Stufe (IX) kann destillativ und/oder über eine Wäsche erfolgen. Erfolgt die Trennung über eine Wäsche und wird gleichzeitig im Verfahren ein Lösungsmittel zum Auswaschen des Isocyanates am Ende der Reaktionsstrecke in der Stufe (VII) verwendet, dann wird als Waschmedium zur Trennung des Chlorwasserstoff/Phosgen-Gemisches bevorzugt dasselbe Lösungsmittel wie zum Auswaschen des Isocyanates eingesetzt.

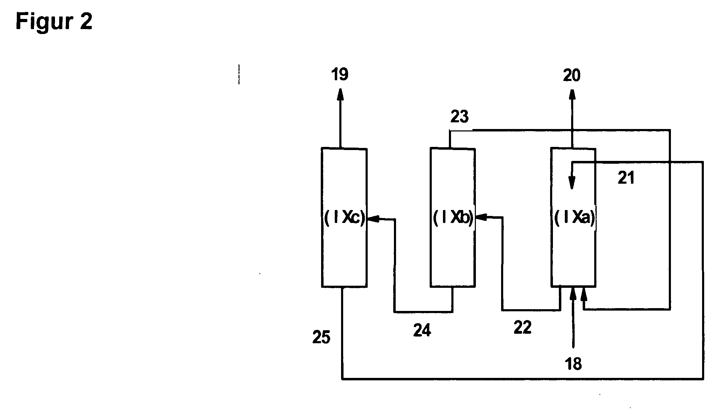

Eine bevorzugte Ausführungsform der Stufe (IX) ist dargestellt in Figur 2.

Darin bedeuten
(IXa) Absorption zur Abtrennung Phosgen
(IXb) Desorption oder Destillation zur Abtrennung von Chlorwasserstoff
(IXc) Desorption oder Destillation zur Auftrennung von Phosgen und Waschflüssigkeit
   - 18: vereinigte gasförmige Ströme
   - 19: Phosgen (rückgeführt)
   - 20: Chlorwasserstoffrückführung
   - 21: Waschflüssigkeit
   - 22: mit Phosgen und gegebenenfalls Chlorwasserstoff beladene Waschflüssigkeit
   - 23: gasförmiger Abzug aus Chlorwasserstoff sowie gegebenenfalls Phosgen
   - 24: von Chlorwasserstoff abgereicherte mit Phosgen beladene Waschflüssigkeit
   - 25: Waschflüssigkeit (rückgeführt)

Die im wesentlichen Phosgen und Chlorwasserstoff sowie gegebenenfalls Lösungsmittel enthaltenden, vereinigten gasförmigen Ströme 18 aus den Stufen (VII) und/oder (VIII) werden bei einem Druck von bevorzugt 1 bis 5 bar in einer Waschvorrichtung (IXa) mit Waschflüssigkeit 21 beaufschlagt. Die Temperatur der Waschflüssigkeit beträgt in der Regel weniger als 10 °C, bevorzugt weniger als 0 °C und besonders bevorzugt weniger als -5 °C. Die Menge an Waschflüssigkeit beträgt die 0,1- bis 5-fache Masse des zugeführten Stroms 18.

Dabei kann es sich um Toluol, Benzol, Nitrobenzol, Anisol, Chlorbenzol, Dichlorbenzol (ortho oder para oder Isomerengemische daraus), Trichlorbenzol, Xylol, Hexan, Diethylisophthalat (DEIP), Tetrahydrofuran (THF), Dimethylformamid (DMF) und deren Gemische, bevorzugt Monochlorbenzol handeln. Besonders bevorzugt wird als Waschflüssigkeit das gleiche Lösungsmittel eingesetzt, wie in Stufe (VII).

Die Wäsche kann im Gleichstrom oder bevorzugt im Gegenstrom erfolgen.

Die mit Phosgen und gegebenenfalls Chlorwasserstoff beladene Waschflüssigkeit 22 wird dann in eine Verfahrenseinheit (IXb) geführt, bei der es sich um eine Desorption oder bevorzugt eine Destillation zur Abtrennung von Chlorwasserstoff aus dieser beladenen Waschflüssigkeit handeln kann. Enthält das Frischphosgen (Strom 5a) signifikante Mengen Chlor (Cl₂), so kann das Frischphosgen ebenfalls in die Stufe (IXb) eingespeist werden, um eine Abtrennung des molekularen Chlors zu erreichen. Der Druck in der Verfahrenseinheit liegt in der Regel um 0,5 - 5 bar über dem in der Stufe (IXa).

Die Trennbedingungen werden bevorzugt so gewählt, daß der Sumpf 24 im wesentlichen lediglich aus Phosgen und Waschflüssigkeit besteht. Der Brüden 23 besteht dagegen im wesentlichen aus Chlorwasserstoff mit geringen Mengen Phosgen und Waschflüssigkeit und wird in Stufe (IXa) zurückgeführt. Der Druck in dieser Einheit entspricht dem in der Stufe (IXb).

Die von Chlorwasserstoff abgereicherte mit Phosgen beladene Waschflüssigkeit 24 wird dann in eine Desorption oder bevorzugt Destillation zur Auftrennung von Phosgen und Waschflüssigkeit (IXc) geführt, in der der im wesentlichen aus Phosgen bestehende Brüdenstrom 19 in die Phosgenierung zurückgeführt wird, wohingegen die Waschflüssigkeit 25, die gegebenenfalls noch untergeordnete Mengen Phosgen enthalten kann, in die Absorption (IXa) rückgeführt wird. Zur Abtrennung von Chlorspuren aus dem Frischphosgen (Strom 4a) kann dieses ebenfalls in die Trennblechkolonne eingespeist werden.

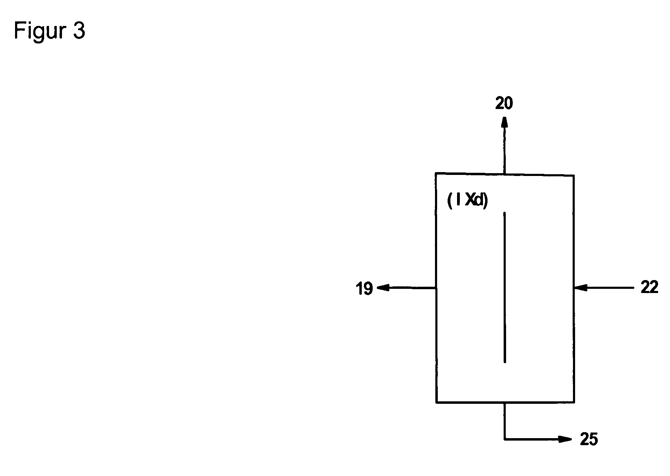

In einer besonders bevorzugten Ausführungsform, die in Figur 3 dargestellt ist, werden dabei die Stufen (IXb) und (IXc) zu einer einzigen Stufe (IXd) vereinigt, bevorzugt handelt es sich dabei um eine Trennblechdestillation, in der die mit Phosgen und gegebenenfalls Chlorwasserstoff beladene Waschflüssigkeit 22 von rechts in die Kolonne geführt wird, so daß Chlorwasserstoff über den Verstärkungsteil als Brüden 20 abgenommen werden kann und die von Chlorwasserstoff abgereicherte mit Phosgen beladene Waschflüssigkeit im Abtriebsteil in die Komponenten getrennt wird.

Aus der linken Kammer wird dann im Seitenabzug 19 gasförmiges Phosgen abgenommen, das über den Abtriebsteil der linken Kammer von der aus dem Sumpf 25 abgenommenen Waschflüssigkeit getrennt wird und über den Verstärkungsteil der linken Kammer vom Chlorwasserstoffbrüden 20 getrennt wird.

Der im erfindungsgemäßen Verfahren abgetrennte Chlorwasserstoffstrom 20 kann weitere leichtsiedende Komponenten enthalten, insbesondere molekulares Chlor (Cl₂) und gasförmige Inerte, wie beispielsweise Stickstoff oder Kohlenstoffmonoxid, aber auch mitgerissene Waschflüssigkeit aus der Stufe (IX). Die Trennblechkolonne wird in der Regel bei einem Druck von 0,5 - 5 bar über dem in der Wascheinheit (IXa) betrieben.

Falls gewünscht können Spuren von Phosgen und/oder bevorzugt Waschflüssigkeit in einer nachgeschalteten Reinigungsstufe (X), beispielsweise einer Schüttung von Aktivkohle, abgetrennt werden.

In dieser nachgeschalteten Reinigungsstufe können etwaige Verunreingungen durch Absorption, Adsorption, Destillation oder Extraktion entfernt werden. Zur Reinigung kann der Chlorwasserstoffstrom auch in Wasser oder verdünnter Salzsäure absorbiert werden und in einem weiteren Schritt nach Abtrennung flüchtiger Bestandteile wieder desorbiert werden. Lösemittelreste können auch durch katalytische Verbrennung in dem Chlorwasserstoffstrom entfernt werden. Der gegebenenfalls so gereinigte Chlorwasserstoffstrom wird der katalytischen Chlorwasserstoff-Oxidation zugeführt.

In einer Ausführungsform des Verfahrens erfolgt die Reinigung des Chlorwasserstoff enthaltenden Stroms durch Überleiten über ein Reinigungsbett und Absorption von darin enthaltenen Lösemittelresten an dem Reinigungsbett.

Das Reinigungsbett besteht aus geeigneten Absorbentien, vorzugsweise in stückiger Form wie Kugeln, Extrudate oder Tabletten. Geeignete Materialien, die als Absorbentien in Frage kommen, sind beispielsweise Aktivkohle, Aluminiumoxid, Titanoxid, Siliziumdioxid, Eisenoxid, Zeolithe und Molsiebe. Geeignete Materialien können auch Metalloxide oder Metallhalogenide, wie Kupfer- oder Rutheniumoxide oder -halogenide bzw. deren Gemische, auf einem Träger aus einem feuerfesten anorganischen Material wie Aluminiumoxid, Titanoxid oder Siliziumdioxid enthalten.

Bevorzugte Absorbentien sind Aluminiumoxid, Aktivkohle und Tonerden.

In einer weitem Ausführungsform des erfindungsgemäßen Verfahrens erfolgt die Reinigung des Chlorwasserstoff enthaltenden Stroms durch katalytische Verbrennung der darin enthaltenen Lösemittelreste. Dazu wird dem Chlorwasserstoff enthaltenden Strom Sauerstoff oder ein Sauerstoff enthaltender Gasstrom, beispielsweise Luft, mit Sauerstoff angereicherte Luft, technischer oder reiner Sauerstoff, zugemischt und dieser Strom über ein Katalysatorfestbett aus Oxidationskatalysator geleitet.

Geeignete Katalysatoren enthalten beispielsweise Aluminiumoxid, Magnesiumoxid, Eisenoxid, Titandioxid, Zirkondioxid oder deren Gemische. Die katalytische Verbrennung der Lösemittelreste (Kohlenwasserstoffe und/oder Chlorkohlenwasserstoffe) an den genannten Katalysatoren kann bereits einen Teilumsatz des enthaltenen ChlorWasserstoffs zum Chlor bewirken.

Dieser Teilumsatz kann beispielsweise bis zu 40%, bevorzugt bis zu 20 %, beispielsweise ca. 5 bis 20% betragen.

Die Durchführung der katalytischen Verbrennung als Reinigungsstufe kann auch als erste Stufe einer zweistufigen katalytischen Chlorwasserstoff-Oxidation betrachtet werden, wobei die erste Stufe an den oben genannten Katalysatoren bis zu einem Teilumsatz und die zweite Stufe (hier: Verfahrensstufe (I)) an den unten beschriebenen Ruthenium enthaltenden Katalysatoren bis zum Vollumsatz, beispielsweise einem Umsatz von mindestens 70%, bezogen auf die erste und die zweite Stufe, durchgeführt wird. Die erste Stufe, die an preiswerten, relativ unempfindlichen Katalysatoren durchgeführt wird, bewirkt eine Oxidation der zu Koksablagerungen führenden Lösemittelspuren zu Kohlendioxid. Dadurch ist der in der zweiten Stufe eingesetzte teure Rutheniumkatalysator vor koksbildenden Verunreinigungen geschützt.

Erfindungswesentlich ist, daß im erfindungsgemäßen Verfahren das in den Verfahrensstufen der eigentlichen Gasphasenphosgenierung, also den Verfahrensstufen (III) bis (IX), befindliche Phosgen im wesentlichen gasförmig vorliegt. Lediglich in zwei Verfahrensstufen, nämlich in der von Stufe (VII) nach Stufe (VIII) ausgetragenen Quenchflüssigkeit sowie in der Stufe (IX) kann Phosgen in kondensierter Form vorliegen.

Durch diese Maßnahme kann der Energieaufwand zur Verdampfung des Phosgen gegenüber den Gasphasenphosgenierungsverfahren aus dem Stand der Technik verringert werden.

Des weiteren beträgt der Quotient aus der in den Verfahrensstufen (III) bis (IX) befindliche Menge an freiem Phosgen und dem Massenstrom an erzeugtem Isocyanat (Strom 17) erfindungsgemäß weniger als 5 h, bevorzugt weniger als 4 h, besonders bevorzugt weniger als 3,5 h und ganz besonders bevorzugt nicht mehr als 3 h.

Dies bedingt eine Senkung des hold-up an Phosgen in den Verfahrensstufen des erfindungsgemäßen Verfahrens gegenüber den Gasphasenphosgenierungsverfahren aus dem Stand der Technik.

Mit der in den Verfahrensstufen befindlichen Menge an Phosgen sind dabei sämtliche Mengen an freiem, d.h. unreagiertem Phosgen bezeichnet, die sich bereits in den Verfahrensstufen (III) bis (IX) befinden, d.h. das in den einzelnen Stufen sowie das in den Leitungen von einer Stufe in die andere befindliche Phosgen inklusive des rückgeführten Phosgen in Leitung 19 (=5).

Ein weiterer Gegenstand der vorliegenden Anmeldung ist ein integriertes Verfahren zur Herstellung von Diisocyanaten, das über die oben beschriebenen Stufen (III) bis (IX), sowie optional (X), noch eine Stufe (I) umfaßt, in der der in dem Gasphasenphosgenierungsverfahren als Kuppelprodukt entstandene Chlorwasserstoff 20 gegebenenfalls nach Durchlaufen der Stufe (X) (Strom 1) in molekulares Chlor (Cl₂) 2 umgewandelt wird sowie eine Phosgenherstellung (II), in der das so gebildete molekulare Chlor 2 mit Kohlenstoffmonoxid 3 in Phosgen überführt wird.

Bei der Chlorherstellung (I) kann es sich beispielsweise um eine Elektrolyse oder bevorzugt um eine Deacon-Verfahren handeln.

Im Deacon-Verfahren erfolgt die katalytische Oxidation des abgetrennten Chlorwasserstoffs mit Sauerstoff zu Chlor.

Dazu werden der gegebenenfalls in einer Stufe (X) gereinigte Chlorwasserstoffstrom, gegebenenfalls ein weiterer Chlorwasserstoff enthaltender Strom und ein Sauerstoff enthaltender Strom in eine Oxidationszone eingespeist und Chlorwasserstoff in Gegenwart eines Katalysators teilweise zu Chlor oxidiert, wobei ein Produktgasstrom erhalten wird, der Chlor, nicht umgesetzten Sauerstoff, nicht umgesetzten Chlorwasserstoff und Wasserdampf enthält.

In diesem auch als Deacon-Prozess bekannten katalytischen Verfahren wird Chlorwasserstoff mit Sauerstoff in einer exothermen Gleichgewichtsreaktion zu Chlor oxidiert, wobei Wasserdampf anfällt.

Übliche Reaktionstemperaturen liegen zwischen 150 und 500°C, übliche Reaktionsdrucke liegen zwischen 1 und 25 bar.

Da es sich um eine Gleichgewichtsreaktion handelt, ist es zweckmäßig, bei möglichst niedrigen Temperaturen zu arbeiten, bei denen der Katalysator noch eine ausreichende Aktivität aufweist. Ferner ist es zweckmäßig, Sauerstoff in überstöchiometrischen Mengen einzusetzen. Üblich ist beispielsweise ein zwei- bis vierfacher Sauerstoff-Überschuss. Da keine Selektivitätsverluste zu befürchten sind, kann es wirtschaftlich vorteilhaft sein, bei relativ hohen Drücken und dementsprechend bei gegenüber Normaldruck längeren Verweilzeiten zu arbeiten.

Geeignete Katalysatoren enthalten Rutheniumoxid, Rutheniumchlorid oder andere Rutheniumverbindungen auf Siliziumdioxid, Aluminiumoxid, Titandioxid oder Zirkondioxid als Träger. Geeignete Katalysatoren können beispielsweise durch Aufbringen von Rutheniumchlorid auf den Träger und anschließendes Trocknen oder Trocknen und Calcinieren erhalten werden. Geeignete Katalysatoren können ergänzend zu oder an Stelle einer Rutheniumverbindung auch Verbindungen anderer Edelmetalle, beispielsweise Gold, Palladium, Platin, Osmium, Iridium, Silber, Kupfer oder Rhenium enthalten. Geeignete Katalysatoren können ferner Chrom(III)oxid enthalten.

Übliche Reaktionsapparate, in denen die katalytische Chlorwasserstoff-Oxidation durchgeführt wird, sind ein Festbett- oder Wirbelbettreaktor. Die Chlorwasserstoff-Oxidation kann mehrstufig durchgeführt werden.

Die katalytische Chlorwasserstoff-Oxidation kann adiabat oder bevorzugt isotherm oder annähernd isotherm, diskontinuierlich, bevorzugt kontinuierlich als Fließ- oder Festbettverfahren, bevorzugt als Festbettverfahren, besonders bevorzugt in Rohrbündelreaktoren an Heterogenkatalysatoren bei Reaktortemperaturen von 180 bis 500°C, bevorzugt 200 bis 400°C, besonders bevorzugt 220 bis 350°C und einem Druck von 1 bis 25 bar, bevorzugt 1,2 bis 20 bar, besonders bevorzugt 1,5 bis 17 bar und insbesondere 2,0 bis 15 bar durchgeführt werden.

Bei der isothermen oder annähernd isothermen Fahrweise können auch mehrere, also 2 bis 10, bevorzugt 2 bis 6, besonders bevorzugt 2 bis 5, insbesondere 2 bis 3 in Reihe geschaltete Reaktoren mit zusätzlicher Zwischenkühlung eingesetzt werden.

Der Sauerstoff kann entweder vollständig zusammen mit dem Chlorwasserstoff vor dem ersten Reaktor oder über die verschiedenen Reaktoren verteilt zugegeben werden. Diese Reihenschaltung einzelner Reaktoren kann auch in einem Apparat zusammengeführt werden.

Eine bevorzugte Ausführungsform besteht darin, dass man eine strukturierte Katalysatorschüttung einsetzt, bei der die Katalysatoraktivität in Strömungsrichtung ansteigt. Eine solche Strukturierung der Katalysatorschüttung kann durch unterschiedliche Tränkung der Katalysatorträger mit Aktivmasse oder durch unterschiedliche Verdünnung des Katalysators mit einem Inertmaterial erfolgen. Als Inertmaterial können beispielsweise Ringe, Zylinder oder Kugeln aus Titandioxid, Zirkondioxid oder deren Gemischen, Aluminiumoxid, Steatit, Keramik, Glas, Graphit oder Edelstahl eingesetzt werden. Beim bevorzugten Einsatz von Katalysatorformkörpern sollte das Inertmaterial bevorzugt ähnliche äußeren Abmessungen haben.

Als Katalysatorformkörper eignen sich beliebige Formen, bevorzugt sind Tabletten, Ringe, Zylinder, Sterne, Wagenräder oder Kugeln, besonders bevorzugt sind Ringe, Zylinder oder Sternstränge.

Als Heterogenkatalysatoren eignen sich insbesondere Rutheniumverbindungen oder Kupferverbindungen auf Trägermaterialen, die auch dotiert sein können, bevorzugt sind gegebenenfalls dotierte Rutheniurnkatalysatoren. Als Trägermaterialen eignen sich beispielsweise Siliciumdioxid, Graphit, Titandioxid mit Rutil- oder Anatas-Struktur, Zirkondioxid, Aluminiumoxid oder deren Gemische, bevorzugt Titandioxid, Zirkondioxid, Aluminiumoxid oder deren Gemische, besonders bevorzugt γ- oder δ-Aluminiumoxid oder deren Gemische.

Die Kupfer- bzw. die Rutheniurnträgerkatalysatoren können beispielsweise durch Tränkung des Trägermaterials mit wässrigen Lösungen von CuCl₂ bzw. RuCl₃ und gegebenenfalls eines Promotors zur Dotierung, bevorzugt in Form ihrer Chloride, erhalten werden. Die Formgebung des Katalysators kann nach oder bevorzugt vor der Tränkung des Trägermaterials erfolgen.

Zur Dotierung eignen sich als Promotoren Alkalimetalle wie Lithium, Natrium, Kalium, Rubidium und Cäsium, bevorzugt Lithium, Natrium und Kalium, besonders bevorzugt Kalium, Erdalkalimetalle wie Magnesium, Calcium, Strontium und Barium, bevorzugt Magnesium und Calcium, besonders bevorzugt Magnesium, Seltenerdmetalle wie Scandium, Yttrium, Lanthan, Cer, Praseodym und Neodym, bevorzugt Scandium, Yttrium, Lanthan und Cer, besonders bevorzugt Lanthan und Cer, oder deren Gemische.

Die Formkörper können anschließend bei Temperaturen von 100 bis 400°C, bevorzugt 100 bis 300°C beispielsweise unter einer Stickstoff-, Argon- oder Luftatmosphäre getrocknet und gegebenenfalls calciniert werden. Bevorzugt werden die Formkörper zunächst bei 100 bis 150°C getrocknet und anschließend bei 200 bis 400°C calciniert.

Der Umsatz an Chlorwasserstoff im einfachen Durchgang kann auf 15 bis 90 %, bevorzugt 40 bis 85 %, besonders bevorzugt 50 bis 70 % begrenzt werden. Nicht umgesetzter Chlorwasserstoff kann nach Abtrennung teilweise oder vollständig in die katalytische Chlorwasserstoff-Oxidation zurückgeführt werden. Das Volumenverhältnis von Chlorwasserstoff zu Sauerstoff am Reaktoreintritt liegt in der Regel zwischen 1:1 und 20: 1, bevorzugt zwischen 2: 1 und 8: 1, besonders bevorzugt zwischen 2: 1 und 5: 1.

Die katalytische Chlorwasserstoff-Oxidation weist gegenüber der Erzeugung von Chlor durch Chlorwasserstoff-Elektrolyse den Vorteil auf, dass keine teure elektrische Energie benötigt wird, dass kein unter Sicherheitsaspekten bedenkliche Wasserstoff als Koppelprodukt anfällt und dass der zugeführte Chlorwasserstoff nicht vollständig rein sein muss.

Die Phosgenherstellung (II) ist bereits oben beschrieben worden.

Die Durchführung der Stufen (I) und (II) ist nicht erfindungswesentlich. Entscheidend ist, daß im erfindungsgemäßen Gasphasenphosgenierungsverfahren der Anteil an kondensiertem bzw. flüssigen Phosgen so gering wie möglich ist.

Ein derartiges integriertes Verfahren kann beispielsweise erfolgen wie beschrieben in WO 2004/014845 oder WO 2004/037718.

Es stellt einen Vorteil eines solchen integrierten Verfahrens dar, daß der als Kuppelprodukt zum Diisocyanat erhaltene Chlorwasserstoff vollständig verwertet werden kann.

### Beispiele

### Beispiel 1: (Vergleich)

Es wird ein Prozess zur Herstellung von TDI (2,4- und 2,6-Toluylendiisocyanat Isomerengemisch) in der Gasphase simuliert. In der Produktionsanlage, bestehend aus den Stufen Reaktion (inklusive Eduktverdampfung, -überhitzung, Reaktion und Quench), Leichtsiederabtrennung, Lösungsmittelabtrennung (mono-Chlorbenzol, MCB) und HCl/Phosgentrennung, wird Phosgen mit TDA (2,4- und 2,6-Toluylendiamin Isomerengemisch) in einem molaren Verhältnis von 8:1 bei 1,9 bar (abs) umgesetzt, unumgesetztes Phosgen destillativ aus dem Reaktionsgemisch abgetrennt, in einer Phosgen/HCl-Trennungsstufe von Chlorwasserstoff abgetrennt und nach Kondensation flüssig in die Reaktionsstufe zur Wiederverwendung zurückgeführt. Frisch zugeführtes Phosgen (19 t/h) wird nach Abtrennung von darin enthaltenem überschüssigem Kohlenmonoxid durch Kondensation dem rückgeführten Phosgen beigemischt. Die Vereinigung der beiden Ströme erfolgt in einem auf -5°C gekühlten Behälter mit einem Fassungsvermögen von 30 m3 (rund 21 t Phosgen bei 50% Füllgrad). Das der Reaktionsstufe zugeführte Phosgen wird verdampft, in einem Überhitzer auf 350°C erhitzt und in einer Mischdüse mit dem Amin vermischt und anschließend zur Reaktion gebracht. Für die Verdampfung des kondensierten Phosgenstroms werden 6,2 MW benötigt.

### Beispiel 2:

Die in Beispiel 1 beschriebene Verschaltung wird erfindungsgemäß so geändert, dass sowohl das frisch zugeführte Phosgen als auch das rückgeführte Phosgen ohne vorherige Zwischenkondensation gasförmig dem Überhitzer zugeführt werden: Dazu wird das rückgeführte Phosgen der Phosgen/HCl-Trennungsstufe gasförmig entnommen. Diesem-Strom wird frisch gasförmig zugeführtes Phosgen aus der Synthesestufe beigemischt. Anschließend wird der nun vereinigte Phosgenstrom überhitzt und dann der Reaktionsstufe zugeführt. Im Unterschied zur der im Beispiel 1 beschriebenen Vorgehensweise fallen Apparate zur Kondensation und der Zwischenbehälter weg, auf die aufwendige Kühlung des Zwischenbehälters kann verzichtet werden. Die Energiekosten für die Kondensation und die Verdampfung des Phosgens entfallen. Zusätzlich wird der Gesamtphosgeninhalt der Anlage um den hold-up des Zwischenbehälters und um das Kondensat in den Rohrleitungen und Wärmetauschern reduziert. Der in Beispiel 1 erforderliche Energiebedarf für Kondensation und Verdampfung des Phosgens entfallen.

## Patentansprüche

1. Verfahren zur Herstellung von Diisocyanaten durch Umsetzung der korrespondierenden Diamine mit Phosgen im stöchiometrischen Überschuß von Phosgen in mindestens einer Reaktionszone, umfassend zumindest die Verfahrensstufen
- Vermischen (V) mindestens eines gasförmiges Phosgen enthaltenden Stromes mit mindestens einem gasförmiges Diamin enthaltenden Strom und Umsetzung dieses Gemisches bei einer Temperatur von 200 bis 600 °C in der mindestens einen Reaktionszone (VI),
- Vermischung (VII) des entstehenden Reaktionsgemischs, das im wesentlichen aus Isocyanat, Phosgen und Chlorwasserstoff besteht, mit mindestens einer Flüssigkeit unter Absenkung der Temperatur auf 100 bis 200 °C, so daß das im Reaktionsgemisch enthaltene Isocyanat durch Kondensation zumindest teilweise in die Flüssigkeit übergeht, während Phosgen und Chlorwasserstoff im wesentlichen vollständig in der Gasphase verbleiben (Quench),
- (VIII) Aufarbeitung des kondensierten Isocyanats unter Verdampfung des in der kondensierten Phase enthaltenen Phosgen und Chlorwasserstoffs,
- (IX) Auftrennung der vereinigten gasförmigen im wesentlichen Chlorwasserstoff und Phosgen enthaltenden Ströme in einen überwiegend Phosgen und einen überwiegend Chlorwasserstoff enthaltenden Strom,
- Rückführung des so erhaltenen überwiegend Phosgen enthaltenden Stromes in die Vermischung (V),
**dadurch gekennzeichnet, daß** das in den Verfahrensstufen befindliche Phosgen zu mindestens 75 Gew% gasförmig vorliegt.

2. Verfahren nach Anspruch 1, **dadurch gekennzeichnet, daß** es sich bei dem Diisocyanat um ein (cyclo)aliphatisches handelt.

3. Verfahren nach Anspruch 1, **dadurch gekennzeichnet, daß** das Diisocyanat ausgewählt aus der Gruppe bestehend aus 1,6-Diisocyanatohexan, 1-Isocyanato-3,3,5- trimethyl-5-(isocyanatomethyl)cyclohexan und 4,4'-Di(isocyanatocyclohexyl)methan.

4. Verfahren nach Anspruch 1, **dadurch gekennzeichnet, daß** es sich bei dem Diisocyanat um ein aromatisches handelt.

5. Verfahren nach Anspruch 4, **dadurch gekennzeichnet, daß** das Diisocyanat ausgewählt ist aus der Gruppe bestehend aus monomerem Methylendi(phenylisocyanat (MDI), 2,4- und/oder 2,6- Toluylendiisocyanat (TDI) und Naphtyldiisocyanat (NDI).

6. Verfahren nach Anspruch 1, **dadurch gekennzeichnet, daß** es sich bei dem isocyanat um Phenylisocyanat handelt.

7. Verfahren nach einem der vorstehenden Ansprüche, **dadurch gekennzeichnet, daß** das in die Stufe (V) geführte Phosgen Kohlenstoffmonoxid enthält.

8. Verfahren nach einem der Ansprüche 1 bis 6, **dadurch gekennzeichnet, daß** das in die Verfahrensstufen geführte Frisch-Phosgen molekulares Chlor (Cl₂) enthält.

9. Verfahren nach Anspruch 8, **dadurch gekennzeichnet, daß** das molekulares Chlor enthaltende Phosgen vor Einspeisung in die Reaktionszone zunächst die Chlorwasserstoffabtrennung (IX) durchläuft.

10. Verfahren nach einem der vorstehenden Ansprüche, **dadurch gekennzeichnet, daß** in der Reaktionszone das molare Verhältnis von Phosgen zu Aminogruppen von 1,1 :1 bis 20 : 1 beträgt.

11. Verfahren nach einem der vorstehenden Ansprüche, **dadurch gekennzeichnet, daß** man die Verfahrensstufe (VII) bei einem Druck von 0,1 bis 20 bar und einer Temperatur von 100 bis 200 °C betreibt.

12. Verfahren nach einem der vorstehenden Ansprüche, **dadurch gekennzeichnet, daß** man die Verfahrensstufe (IX) so betreibt, daß der Massenbruch von Chlorwasserstoff im phosgenhaltigen Strom nach gegebenenfalls durchgeführter Vermischung mit Frisch-Phosgen vor der Vermischung mit dem aminhaltigen Strom weniger als 15 Gew.-% beträgt.

13. Verfahren nach einem der vorstehenden Ansprüche, **dadurch gekennzeichnet, daß** der Quotient aus der in den Verfahrensstufen (III) bis (IX) befindliche Menge an freiem Phosgen und dem Massenstrom an erzeugtem Isocyanat (Strom 17) weniger als 5 h beträgt.

## Claims

1. A process for preparing diisocyanates by reacting the corresponding diamines with phosgene in a stoichiometric excess of phosgene in at least one reaction zone, comprising at least the process stages of:
- mixing (V) at least one stream comprising gaseous phosgene with at least one stream comprising gaseous diamine, and converting this mixture at a temperature of from 200 to 600°C in the at least one reaction zone (VI),
- mixing (VII) the resulting reaction mixture, which consists essentially of isocyanate, phosgene and hydrogen chloride, with at least one liquid while lowering the temperature to from 100 to 200°C, such that the isocyanate present in the reaction mixture is transferred at least partly to the liquid by condensation, while phosgene and hydrogen chloride remain essentially completely in the gas phase (quench),
- (VIII) working up the condensed isocyanate with evaporation of the phosgene and hydrogen chloride present in the condensed phase,
- (IX) separating the combined gaseous streams comprising essentially hydrogen chloride and phosgene into a stream comprising predominantly phosgene and a stream comprising predominantly hydrogen chloride,
- recycling the thus obtained stream comprising predominantly phosgene into the mixing (V), wherein the phosgene present in the process stages is present in gaseous form to an extent of at least 75% by weight.

2. The process according to claim 1, wherein the diisocyanate is a (cyclo)aliphatic diisocyanate.

3. The process according to claim 1, wherein the diisocyanate is selected from the group consisting of 1,6-diisocyanatohexane, 1-isocyanato-3,3,5-trimethyl-5-(isocyanatomethyl)cyclohexane and 4,4'-di(isocyanatocyclohexyl)methane.

4. The process according to claim 1, wherein the diisocyanate is an aromatic diisocyanate.

5. The process according to claim 4, wherein the diisocyanate is selected from the group consisting of monomeric methylenedi(phenyl isocyanate) (MDI), tolylene 2,4- and/or 2,6-diisocyanate (TDI) and naphthyl diisocyanate (NDI).

6. The process according to claim 1, wherein the isocyanate is phenyl isocyanate.

7. The process according to any of the preceding claims, wherein the phosgene conducted into stage (V) comprises carbon monoxide.

8. The process according to any of claims 1 to 6, wherein the fresh phosgene conducted into the process stages comprises molecular chlorine (Cl₂).

9. The process according to claim 8, wherein the phosgene comprising molecular chlorine, before being fed into the reaction zone, first passes through the hydrogen chloride removal (IX).

10. The process according to any of the preceding claims, wherein the molar ratio of phosgene to amino groups in the reaction zone is from 1.1 : 1 to 20 : 1.

11. The process according to any of the preceding claims, wherein process stage (VII) is operated at a pressure of from 0.1 to 20 bar and a temperature of from 100 to 200°C.

12. The process according to any of the preceding claims, wherein process stage (IX) is operated such that the mass fraction of hydrogen chloride in the phosgene-containing stream, after optional mixing with fresh phosgene before the mixing with the amine-containing stream, is less than 15% by weight.

13. The process according to any of the preceding claims, wherein the quotient of the amount of free phosgene present in process stages (III) to (IX) and the mass flow of isocyanate formed (stream 17) is less than 5 h.

## Revendications

1. Procédé pour la préparation de diisocyanates par transformation des diamines correspondantes avec du phosgène, avec un excès stoechiométrique de phosgène dans au moins une zone de réaction, comprenant au moins les étapes de procédé
- mélange (V) d'au moins un flux gazeux contenant du phosgène avec au moins un flux gazeux contenant une diamine et transformation de ce mélange à une température de 200 à 600°C dans au moins une zone de réaction (VI),
- mélange (VII) du mélange réactionnel formé, qui est essentiellement constitué d'isocyanate, de phosgène et chlorure d'hydrogène, avec au moins un liquide avec abaissement de la température à 100 jusqu'à 200°C, de manière telle que l'isocyanate contenu dans le mélange réactionnel passe au moins partiellement par condensation dans le liquide, alors que le phosgène et le chlorure d'hydrogène restent de manière essentiellement complète dans la phase gazeuse (Quench),
- (VIII) traitement de l'isocyanate condensé avec évaporation du phosgène et du chlorure d'hydrogène contenus dans la phase condensée,
- (IX) séparation des flux gazeux rassemblés contenant essentiellement du chlorure d'hydrogène et du phosgène en un flux contenant principalement du phosgène et un flux contenant principalement du chlorure d'hydrogène,
- recyclage du flux contenant principalement du phosgène ainsi obtenu dans le mélange (V),
**caractérisé en ce que** le phosgène se trouvant dans les étapes de procédé se trouve à raison d'au moins 75% en poids sous forme gazeuse.

2. Procédé selon la revendication 1, **caractérisé en ce qu'**il s'agit, pour le diisocyanate, d'un diisocyanate (cyclo)aliphatique.

3. Procédé selon la revendication 1, **caractérisé en ce que** le diisocyanate est choisi dans le groupe constitué par le 1,6-diisocyanatohexane, le 1-isocyanato-3,3,5-triméthyl-5-(isocyanatométhyl)cyclohexane et le 4,4'-di(isocyanatocyclohexyl)méthane.

4. Procédé selon la revendication 1, **caractérisé en ce qu'**il s'agit, pour le diisocyanate, d'un diisocyanate aromatique.

5. Procédé selon la revendication 4, **caractérisé en ce que** le diisocyanate est choisi dans le groupe constitué par le méthylènedi(phénylisocyanate) (MDI), le 2,4-toluylènediisocyanate et/ou le 2,6-toluylènediisocyanate (TDI) et le naphtyldiisocyanate (NDI) monomères.

6. Procédé selon la revendication 1, **caractérisé en ce qu'**il s'agit, pour l'isocyanate, de phénylisocyanate.

7. Procédé selon l'une quelconque des revendications précédentes, **caractérisé en ce que** le phosgène guidé dans l'étape (V) contient du monoxyde de carbone.

8. Procédé selon l'une quelconque des revendications 1 à 6, **caractérisé en ce que** le phosgène frais guidé dans les étapes de procédé contient du chlore moléculaire (Cl₂).

9. Procédé selon la revendication 8, **caractérisé en ce que** le phosgène contenant du chlore moléculaire passe, avant son injection dans la zone de réaction, d'abord dans une étape de séparation du chlorure d'hydrogène (IX).

10. Procédé selon l'une quelconque des revendications précédentes, **caractérisé en ce que** dans la zone de réaction, le rapport molaire de phosgène aux groupes amino est de 1,1:1 à 20:1.

11. Procédé selon l'une quelconque des revendications précédentes, **caractérisé en ce qu'**on réalise l'étape de procédé (VII) à une pression de 0,1 à 20 bars et à une température de 100 à 200°C.

12. Procédé selon l'une quelconque des revendications précédentes, **caractérisé en ce qu'**on réalise l'étape de procédé (IX) de manière telle que la fraction massique de chlorure d'hydrogène dans le flux contenant du phosgène après le mélange le cas échéant réalisé avec du phosgène frais, avant le mélange avec le flux contenant l'amine, est inférieure à 15% en poids.

13. Procédé selon l'une quelconque des revendications précédentes, **caractérisé en ce que** le quotient de la quantité de phosgène libre se trouvant dans les étapes de procédé (III) à (IX) et du flux massique d'isocyanate généré (flux 17) est inférieur à 5 h.
